(19) Europäisches Patentamt
European Patent Office
Office européen des brevets

(11) Publication number : **0 538 127 A1**

(12) **EUROPEAN PATENT APPLICATION**

(21) Application number : **92402812.9**

(22) Date of filing : **14.10.92**

(51) Int. Cl.$^5$ : **C08F 297/02,** C09D 153/00, A61L 33/00

(30) Priority : **14.10.91 JP 264171/91**

(43) Date of publication of application : **21.04.93 Bulletin 93/16**

(84) Designated Contracting States : **BE DE FR GB IT NL SE**

(71) Applicant : **TERUMO Kabushiki Kaisha**
**44-1 Hatagaya 2-chome Shibuya-ku**
**Tokyo (JP)**

(72) Inventor : **Senshu, Kazuhisa, c/o Terumo K.K.**
**1500, Inokuchi, Nakai-machi**
**Ashigarakami-gun, Kanagawa-ken (JP)**
Inventor : **Nakahama, Seiichi**
**48-17, Sakuradai, Midori-ku**
**Yokohama-shi, kanagawa-ken (JP)**

(74) Representative : **Gillard, Marie-Louise et al**
**Cabinet Beau de Loménie 158, rue de l'Université**
**F-75340 Paris Cédex 07 (FR)**

(54) **Medical materials having blood compatibility.**

(57) A block copolymer which comprises recurring units (a) and (b) and/or (c) represented by the following general formulae :

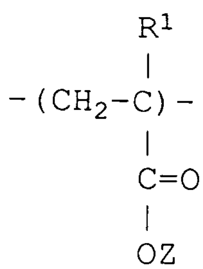
(a),

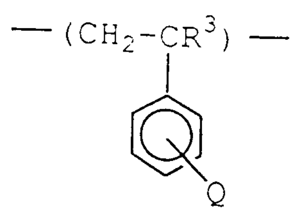
(b),

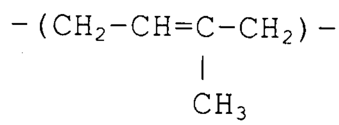
(c)

wherein $R^1$ is H, an alkyl group or a cyano group, Z is $-R^2OH$, $R^2$ is an alkylene group, $R^3$ is H or a methyl group and Q is an alkyl group.

By the use of the block copolymer of this invention, a medical device is provided which has a lamellar, cylindrical or sea-island microphase separated structure on its surface and has excellent blood compatibility and flexibility.

EP 0 538 127 A1

## FIELD OF THE INVENTION

This invention relates to medical materials having blood compatibility. More particularly, it relates to block copolymers having high blood compatibility and flexibility, in which hydrophilic segments and specified hydrophobic segments in one molecule are linked one another in controlled manner. It also relates to a medical material to which the inventive block copolymer is applied.

## BACKGROUND OF THE INVENTION

Polyhydroxy acrylates and polyhydroxy methacrylates have affinity for water due to the presence of hydroxyl groups on their side chains, but within a limited range because of the high hydrophobic nature of their backbones. Poly(2-hydroxyethyl methacrylate), known as Hydron, is a typical example of such polymers. It contains in its molecule not only hydrophobic $\alpha$-methyl groups but also hydrophilic hydroxyl groups located on distant portions from the backbone and principal chain, thus showing both hydrophilic and hydrophobic properties.

Because of such nature, the just described polymer is capable of showing biological compatibility and therefore has been regarded as a favorable material in the field of synthesized polymers for medical use. Its monomer, however, is apt to cause three dimensional reaction at the time of polymerization due to the presence of irreversibly formed diester which is a monomer having two double bonds, thus frequently causing gelation and entailing a difficulty in obtaining a chain form polymer. Such a phenomenon is undesirable in the extreme from a viewpoint of designing a polymer having a controlled molecular chain length. With the aim of overcoming such a problem, processes have been developed in which a hydrophilic segment and a hydrophobic segment are blended or subjected to random copolymerization to obtain a material having both hydrophilic and hydrophobic properties. These processes, however, can bring out merely a property which corresponds to the compositional ratio of two different polymers or of hydrophilic and hydrophobic block units. On the contrary, a block copolymer comprises a hydrophilic segment and a hydrophobic segment. These different hydrophilic and hydrophobic blocks are linked in one molecule and can form micro domains at the molecular assembly level. Because of this, it is possible to add,a new function to such a block copolymer.

A typical example of high molecular compounds in which hydrophilic and hydrophobic segments are arranged in one molecule in a controlled state is a block copolymer which is obtained by coupling a semi-telechelic oligomer having an amino group on one end of the chain with a telechelic oligomer having isocyanate groups on both ends. Such a type of block copolymer, however, has disadvantages in that the procedure for its synthesis is complex, its yield by the coupling reaction is extremely poor and purification of the formed block copolymer is unusually difficult. Also, since radical polymerization is employed, molecular weight distribution of each of the formed oligomers is very wide, thus entailing a difficulty in controlling molecular weight of the oligomer. Also, it is highly possible that a portion of the oligomer remains unreacted and causes a difficulty in purifying the product. In addition, since a urea bond is present between the hydrophilic and hydrophobic segments, hydrogen bonding strength between the obtained block copolymer molecules becomes large which causes a difficulty in controlling a microphase separation structure, as well as a poor affinity for solvent because of the high hydrogen bonding strength between urea bonds.

With the recent advance in anionic living polymerization techniques, it became possible to synthesize block copolymers having various structures with high monodispersibility even in the absence of a dispersing agent. However, in the case of a vinyl monomer having a reactive group in its molecule, such as 2-hydroxyethyl methacrylate, a transfer reaction is generated due to active hydrogen in the reactive group, thus entailing deactivation of the anionic living activity. Because of this, anionic living polymers cannot be obtained directly from such vinyl monomers.

It has been found, however, that an anionic living polymer can be obtained from a vinyl monomer which is protected with a substituted silyl group, by protecting a reactive group such as hydroxyl group in advance with a substituted silyl group and then subjecting the thus protected monomer to anionic living polymerization, and that the substituted silyl group as a protective group can be left easily from the thus obtained polymer by contacting it with a proton donor such as methanol, ethanol, hydrochloric acid, sulfuric acid or the like. Thus, it is now possible to obtain anionic living polymers from acrylic acid based compounds.

## SUMMARY OF THE INVENTION

An object of the present invention is to provide a block copolymer in which, unlike the case of a copolymer consisting of randomly linked hydrophilic and hydrophobic monomers, hydrophilic and hydrophobic monomers separately form blocks, that is, a block copolymer which is obtained by linking hydrophilic and hydrophobic

segments that can respectively form hydrophilic and hydrophobic domains in a solution not at a molecular level but at a molecular assembly level.

Another object of the present invention is to provide a block copolymer capable of exhibiting blood compatibility in the living body, which, since the blood compatibility is expressed by the formation of a microphase separated structure from hydrophilic and hydrophobic domains, has a lamellar or a cylindrical microphase separation structure that can be observed under a transmission electron microscope.

Still another object of the present invention is to provide a block copolymer which has a high flexibility resulting from the conversion of hydrophobic domain-constituting hydrophobic monomers in the block copolymer into a structure having side chains of a substituted benzene ring or isoprene monomers and which is suitable for use in a complex material with other material.

A further object of the present invention is to provide a medical material having blood compatibility, such as an artificial blood vessel, an artificial skin, a catheter, a blood bag, an artificial lung or the like, to which the inventive block copolymer is applied singly or as a complex material with other material.

Other objects and advantages of the present invention will be made apparent as the description progresses.

## BRIEF DESCRIPTION OF THE DRAWINGS

Fig. 1 is a scanning electron micrography showing the surface of a block copolymer obtained in Comparative Example 3 after its bending test.

Fig. 2 is a scanning electron micrography showing the surface of a block copolymer obtained in Inventive Example 1 after its bending test.

Fig. 3 is a scanning electron micrography showing the surface of a block copolymer obtained in Inventive Example 4 after its bending test.

## DETAILED DESCRIPTION OF THE INVENTION

The aforementioned objects can be solved by a medical material having blood compatibility, prepared from a block copolymer which comprises:
a repeating unit (a) represented by the following general formula

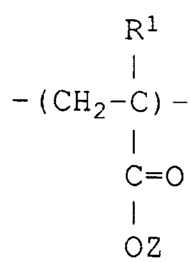

(a)

wherein $R^1$ is a hydrogen atom, an alkyl group having 1 to 4 carbon atoms or a cyano group, Z is $-R^2OH$, and $R^2$ is an alkylene group having 1 to 6 carbon atoms;
a repeating unit (b) represented by the following general formula

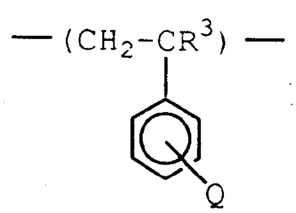

(b)

wherein $R^3$ is a hydrogen atom or a methyl group and Q is an alkyl group having 1 to 8 carbon atoms; and/or
a repeating unit (c) represented by the following general formula

$$-(CH_2-CH=\underset{\displaystyle CH_3}{\underset{|}{C}}-CH_2)- \qquad (c)$$

In the above formulae, $R^1$ is preferably a hydrogen atom or a methyl group, more preferably a methyl group; Z is $R^2OH$ where $R^2$ is preferably an alkylene group having 2 to 4 carbon atoms, more preferably an ethylene group; and Q is preferably an octyl group.

The recurring units (b) and (c) may be used alone or as a mixture to form a copolymer with the recurring unit (a), or may be used together with other compound to change physical properties of the resulting material such as flexibility. For example, the copolymer with n-butyl methacrylate has properties of increased softness and the copolymer with $\alpha$-methyl styrene has properties of increased hydrophobic.

Though not particularly limited, the copolymer of the present invention may be produced preferably by means of anionic living polymerization.

Synthesis of a block copolymer from telechelic oligomers by means of coupling is extremely difficult and will entail a poor yield by the coupling reaction and cause a difficulty in purifying the formed block copolymer. Radical polymerization will entail extremely broad molecular weight distribution of each resulting oligomer, thus causing a difficulty in controlling molecular weight of the oligomer, in addition to a possibility of the presence of unreacted oligomers which will cause elution and the like problems.

When the block copolymer to be used in the present invention is obtained by anionic living polymerization, various effects can be obtained which cannot be found in a block copolymer prepared by coupling reaction of telechelic oligomers. For example, it can be synthesized easily, its reaction efficiency is markedly high with a yield of almost 100%, it can be purified easily because of almost no remaining monomers and it has a markedly narrow molecular weight distribution which renders possible easy molecular weight control of the block copolymer. For example, the ratios (Mw/Mn) of weight-average molecular weight (Mw) to number-average molecular weight (Mn) are up to 1.2. In addition, since a hydrophilic segment and a hydrophobic segment are linked each other directly, a microphase separated structure can be obtained easily by controlling the reaction. Also, its molecular weight, molecular chain length and molecular chain length ratio can be controlled as designed, and it can be synthesized in a large quantity.

A living block copolymer consisting of a hydrophilic segment (A) and a hydrophobic segment (B) or another hydrophobic block (B′) and having a sequence of for example B-A, A-B-A, A-B-B′-A or the like can be obtained by employing anionic living polymerization. Such a copolymer may have a number average molecular weight of generally from 500 to 100,000, preferably from 1,000 to 50,000, more preferably from 7,000 to 30,000. The number average molecular weight if smaller than 500 would cause solubilization of the copolymer, thus entailing a difficulty in treating it on the surface of a base material, and if larger than 100,000 would reduce kinetic capacity of the molecular chain and therefore would cause a difficulty in coating the resulting copolymer to the surface of a base material.

The hydrophilic segment (A) consisting of the recurring unit (a) of the present invention and the hydrophobic segment (B) consisting of the recurring unit (b) and/or (c) may be used in any ratio in the copolymer, but the recurring unit molar ratio (A)/(B) may be generally 5-95/95-5, preferably 30-70/70-30, more preferably 40-60/60-40. Ratio of the hydrophilic segment (A) if smaller than 5:95 would cause decreased ratio of hydrophilic moiety on the surface of a base material and therefore would bear no microphase separated structure, and if larger than 95:5 would result in too high ratio of hydrophilic moiety on the surface of a base material and therefore would bear no microphase separated structure.

Preferably, the number of the recurring unit (a) (i.e., the degree of polymerization) in the hydrophilic segment may be 10 or more, and the number of the recurring unit (b) and/or (c) in the hydrophobic segment may be 10 or more. The number of recurring units if smaller than this level would bear no suitable microphase separated structure.

Since the state of molecules of these block copolymer in a solution generally varies greatly depending on the property of each solvent to be used, it is possible to control assembling conditions of blocks by charging affinity of solvent for each block. In consequence, a microphase separated structure having optional hydrophilic and hydrophobic properties can be realized by dissolving these block copolymers in a properly selected solvent and making the solution into films by casting, even when these block copolymers have the same molecular structure. Also, it is possible to change the phase separated structure by changing the casting temperature or by treating the cast film with heat (annealing).

In addition, the microphase separated structure can be controlled within a wide range by the use of various types of block copolymers having different molecular structures. For example, it is possible to change the phase

separation structure of a hydrophilic block (A) and a hydrophobic block (B) from a sea-island structure to a lamellar structure by changing the recurring unit molar fraction of (A) and (B). It is possible also to control domain width of a lamellar phase separation structure by changing molecular weights of the hydrophilic and hydrophobic blocks (A) and (B). In other words, a microphase separation structure having both hydrophilic and hydrophobic properties can be controlled in the block copolymer of the present invention, which cannot be attained in a blend polymer. For example, in the case of an HEMA-OctSt-HEMA copolymer consisting of 2-hydroxyethyl methacrylate (HEMA) as the hydrophilic segment (A) and octyl styrene (OctSt) as the hydrophobic segment (B) and having an HEMA molar fraction of 0.5 and a total number average molecular weight of 23,800, lamellar structure of a film obtained by casting a solution of the copolymer on a base material showed a domain width of 9 nm when dimethylformamide (DMF) was used as a solvent or 8 nm when a mixture consisting of tetrahydrofuran (THF) and methanol (9/1) was used as a solvent.

The block copolymer surface having such a microphase separation structure shows excellent biological compatibility which cannot be found in homopolymers or random copolymers. Such an effect is remarkable in a lamellar structure having both hydrophilic and hydrophobic properties and in an island structure in which the hydrophilic property is regarded as the sea, especially the domain width is in the range of from 5 to 500 nm.

Since such a block copolymer is highly transparent and can be dissolved in solvents, it can be molded into various shapes by casting or spreading a solution of the copolymer in or on a pattern or mold having a desired shape such as a film, a plate, a tube or the like and subsequently removing the solvent. Microphase separated structure of such a molded product can be controlled by selecting proper solvent or temperature at the time of the molding of the block copolymer.

The block copolymer of the present invention is obtained preferably by means of anionic living polymerization which is effective in producing a block copolymer having a markedly narrow molecular weight distribution. Molecular weight of the hydrophobic block is determined based on the type of an initiator and the concentration ratio of the initiator to a first monomer (a hydrophobic forming monomer), and molecular weight of the hydrophilic block is determined based on the amount of a subsequently added second monomer (a hydrophilic forming monomer). Recurring mode of the blocks (A-B, A-B-A and the like), molecular weight, block length, block length ratio and the like can also be changed. Illustrative examples of the solvent include tetrahydrofuran, toluene, hexane and the like, and those of the initiator include n-butyllithium, naphthalene lithium salt, naphthalene sodium salt and the like. The use of naphthalene lithium salt as an initiator results in the formation of an A-B-A type block copolymer, and the use of n-butyllithium results in the formation of an A-B type block copolymer. Unlike the case of usual polymers having hydrophilic and hydrophobic properties, these block copolymers can form hydrophilic and hydrophobic blocks within a single molecule level. Because of this, the block copolymer of the present invention can be used as a material having biological compatibility.

It is desirable to use the block copolymer of the present invention by coating it on the surface of a medical device which contacts with blood, such as a catheter. Coating of the block copolymer of the present invention may be effected by applying its solution to the surface of a medical device of interest and then drying the applied solution to form a film layer, or by dipping the device in the solution and then drying the device. The film layer thus coated has a high flexibility and does not show cracks which are formed frequently in the case of the prior art materials. The coated film layer when dried may have a thickness of 0.1 μm or more, preferably in the range of from 1 to 500 μm. The film thickness if smaller than 0.1 μm would bear no biological compatibility and if too large would exert a bad influence on the physical properties of a medical device to be used as a base material.

## EXAMPLES

The following examples are provided to further illustrate the present invention. It is to be understood, however, that the examples are for purpose of illustration only and are not intended as a definition of the limits of the invention.

### Example 1

The block copolymer of the present invention was synthesized making use of the anionic living polymerization technique. Experiments were carried out in a highly vacuum atmosphere by employing a breakable seal method.

A 0.563 mmol portion of Li-naphthalene as an initiator and 30.9 mmol of octyl styrene (OctSt) as a hydrophobic repeating unit were dissolved in 100 ml of tetrahydrofuran (THF), and the resulting solution (final concentration, 0.31 mol/liter) was stirred at -78°C for 1 hour to effect anionic living polymerization. After the polymerisation of octyl styrene, diphenylethylene (DPE, 1.2 mmol) as a capping agent was added and reacted for 10 min. Moreover, to the thus obtained living polymer solution was added 26.4 mmol of 2-hydroxyethyl methacrylate (HEMA) whose hydroxyl group has been protected with trimethylsilyl group. The thus prepared mixture was stirred at -78°C for 1 hour to prepare a living block copolymer which was subsequently treated with a 1.5 N HCl/methanol solution to effect deprotection reaction, thereby obtaining an HEMA-OctSt-HEMA type block copolymer. Identification of the thus obtained block copolymer was carried out by nuclear magnetic resonance (NMR) analysis and the like means. Structure and the number of average recurring units of the thus obtained block copolymer are shown in Table 1.

## Example 2 to 4

Block copolymers having different block chain lengths were obtained by repeating the process of Example 1 except that different initiators were used as shown in Table 1. Structure and the number of average repeating units of each block copolymer thus obtained are also shown in Table 1.

Since the polymerization degree of each block copolymer is expressed as the number of repeating units in Table 1, number average molecular weight of the copolymer can be calculated by multiplying the number of each repeating unit by its molecular weight.

Table 1

| Ex. No. | Initiator | Structure | Average recurring unit No. | Morphology and domain width (nm) | Solvent used for casting | Mw/Mn |
|---|---|---|---|---|---|---|
| 1 | LiNaph | HEMA-OctSt-HEMA | 47-33-47 | lamella, 15 | DMF | 1.06 |
| 2 | BuLi | HEMA-OctSt | 44-32 | lamella, 20 | DMF | 1.07 |
| 3 | BuLi | HEMA-Isp | 37-77 | lamella, 35 | THF | 1.14 |
| 4 | LiNaph | HEMA-Isp-HEMA | 50-108-50 | lamella, 25 | THF | 1.05 |

Note: LiNaph, naphthalenelithium; BuLi, n-butyllithium; HEMA, 2-hydroxyethyl methacrylate; OctSt, octyl styrene; Isp, isoprene

## Evaluation tests

In order to confirm formation of microphase separation structure, each of the block copolymers obtained in Examples 1 to 4 was dissolved in respective solvent shown in Table 1, and the solution was spread on a Teflon plate. Each of the cast films thus prepared was made into ultra thin sections in the usual way to observe microphase separated structure under a transmission electron microscope (TEM). All of the block copolymers obtained in Examples 1 to 4 showed formation of a lamellar microphase separated structure. Molecular weight distributions (Mw/Mn) of these block copolymers were determined by gel permeation chromatography (GPC) as shown in Table 1.

Each of the block copolymers obtained in Examples 1 to 4 was made into 3% DMF solution. A commercially available polyurethane sheet was dipped into the thus prepared solution and then dried to obtain a sheet coated with the block copolymer. Each of the thus coated sheets was contacted for 30 minutes with platelet rich plasma (100,000 platelets/μl) which has been obtained from a blood sample collected from a healthy person. In this way, blood compatibility of the inventive block copolymer was evaluated. As Comparative Examples 1 and 2, a polyurethane sheet and a polypropylene sheet were prepared and evaluated in the same manner as described above. Results of the platelet adhesion test are shown in Table 2.

Table 2

| Examples | Number of adhered platelets | Morphology of platelets |
|---|---|---|
| Inventive Ex. 1 | few | disk |
| Inventive Ex. 2 | few | disk |
| Inventive Ex. 3 | few | disk |
| Inventive Ex. 4 | few | disk |
| Comparative Ex. 1 | many | spherical, partly forming a pseudopodium |
| Comparative Ex. 2 | many | no trace of the original form |

In order to evaluate flexibility of the block copolymers obtained in Examples 1 and 4, each of the copolymers was made into 3% DMF solution. As a reference example (Comparative Example 3), a block copolymer of HEMA-St-HEMA type (45-114-45 in average recurring unit numbers) having a cylindrical microphase separation structure with a domain width of 11 nm was prepared by repeating the process of Example 1 except that styrene was used instead of octyl styrene, and the thus obtained block copolymer was also made into 3% DMF solution. Inner and outer surfaces of a polyurethane tube having an inner diameter of 3 mm were coated with each block copolymer by dipping the tube into respective DMF solution and then drying the thus treated tube. Each of the thus coated tubes was subjected to bending test by deforming it into a loop of about 1 cm. Thereafter, inner and outer surfaces of each tube were observed under a scanning electron microscope (SEM) to examine the presence of cracks and the like on the surface. The results are shown in the following table. SEM photographs are also shown in Figs. 1 to 3.

| | Samples | Inner surface of tube | Outer surface of tube | Fig. |
|---|---|---|---|---|
| CE3 | HEMA-St-HEMA | cracks in large number | cracks in large number | 1 |
| EX1 | HEMA-OctSt-HEMA | no cracks | no cracks | 2 |
| EX4 | HEMA-Isp-HEMA | no cracks | no cracks | 3 |

Example 5

As an example of an industrial application of this invention, an artificial blood vessel having an inner diameter of 3 mm and a length of 7 cm which consists of an outer layer made of Dacron® (a polyester knitted article manufactured by Du Pont), an intermediate layer made of polyurethane coating intended to smoothen the surface of the lumen of Dacron® and an inner layer made of a block copolymer of the present invention was prepared, and incorporated into both carotid arteries of a mongrel dog. When the artificial blood vessel was taken out from the dog 7, 14, 30, 92 and 372 days after, patency was seen in all cases, and no thrombus was observed on the surface of the lumen of the blood vessel through a scanning electron microscope.

As has been described in the foregoing, the block copolymer of the present invention which comprises a hydrophilic block and a specified hydrophobic block is possessed of low platelete adhesion and high flexibility due to the formation of a lamellar or the like microphase separation structure on its surface. Because of such excellent properties, the inventive block copolymer is best suited for use in medical devices which have blood-contacting portions.

Also, though the block copolymer of the present invention can be made into a sheet or the like as it is, a medical device having blood compatibility with no cracks or the like on its coat layer can be obtained by coating the block copolymer of the present invention on the surface of other sheet, tube, thread or the like material. For example, a tube coated with the inventive block copolymer can be applied to various catheters, artificial blood vessel and the like, a sheet coated with the block copolymer can be applied to artificial skin, and a thread to surgical suture.

## Claims

1. A block copolymer which comprises:
a recurring unit (a) represented by the following general formula

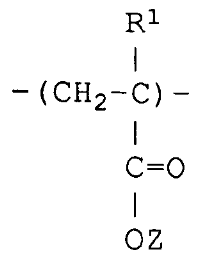

(a)

wherein $R^1$ is a hydrogen atom, an alkyl group having 1 to 4 carbon atoms or a cyano group, Z is $-R^2OH$, and $R^2$ is an alkylene group having 1 to 6 carbon atoms;
a recurring unit (b) represented by the following general formula

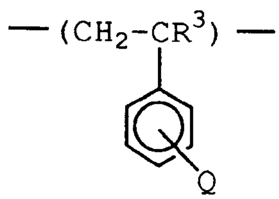

(b)

wherein $R^3$ is a hydrogen atom or a methyl group and Q is an alkyl group having 1 to 8 carbon atoms; and/or
a repeating unit (c) represented by the following general formula

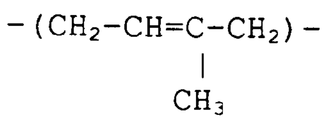

(c)

2. The block copolymer according to claim 1 wherein said block copolymer is obtained by anionic living polymerization.

3. The block copolymer according to claim 1 wherein hydrophilic and hydrophobic blocks have a microphase separated structure.

4. The block copolymer according to claim 1 wherein at least 10 of said recurring unit (a) form a chain and at least 10 of said recurring unit (b) and/or (c) form another chain in one molecule.

5. The block copolymer according to claim 1 wherein a ratio of weight-average molecular weight to number-average molecular weight is up to 1.2.

6. A catheter of a polyolefin base in which the surface of said polyolefin base has a coat layer that comprises the block copolymer of claim 1.

FIG. 1

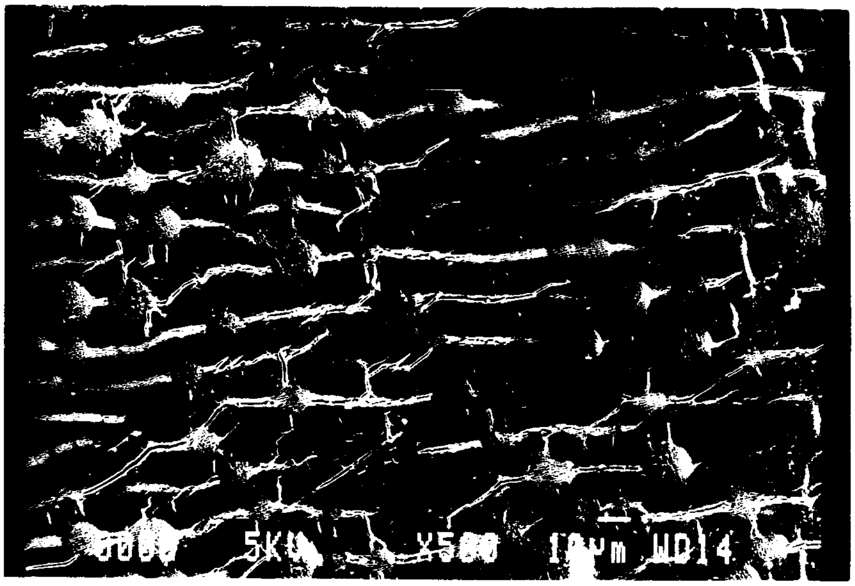


FIG. 2

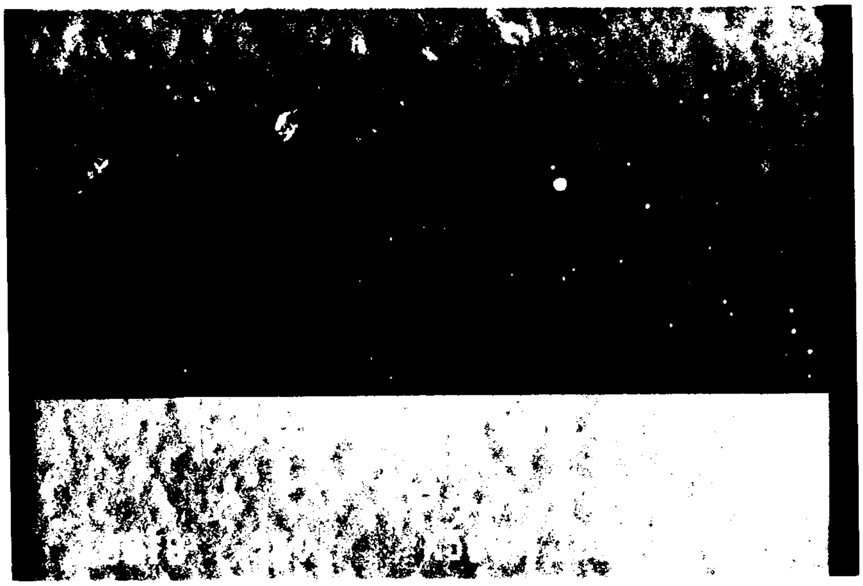

FIG. 3

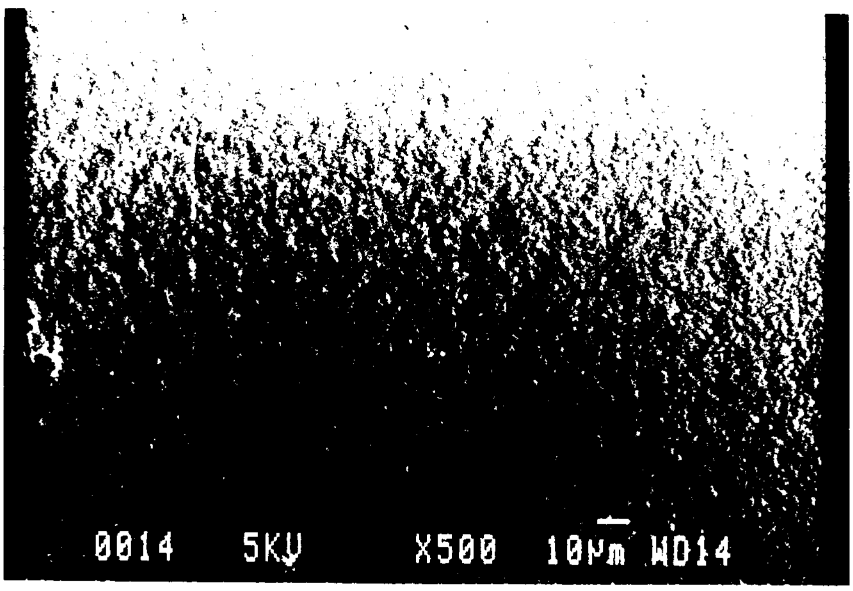

European Patent Office

# EUROPEAN SEARCH REPORT

Application Number

EP 92 40 2812

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X<br>Y | EP-A-0 296 850 (MITSUBISHI PETROCHEMICAL CO.)<br>* Preparation 1 *<br>* claims 1,3,11 * | 1<br>1-6 | C08F297/02<br>C09D153/00<br>A61L33/00 |
| Y | DATABASE WPIL<br>Week 8847,<br>Derwent Publications Ltd., London, GB;<br>AN 88-333767<br>& JP-A-63 246 170 (NIPPON ZEON K.K.) 13 October 1988<br>* abstract * | 1-6 | |
| A | EP-A-0 323 341 (TERUMO K.K.)<br>* claims 7,9,14 * | 1-6 | |
| A | DATABASE WPIL<br>Week 8514,<br>Derwent Publications Ltd., London, GB;<br>AN 85-083894<br>& JP-A-60 034 451 (TERUMO CORP.) 22 February 1985<br>* abstract * | 1-6 | |
| | | | TECHNICAL FIELDS SEARCHED (Int. Cl.5) |
| | | | C08F<br>C09D<br>A61L |

The present search report has been drawn up for all claims

| Place of search | Date of completion of the search | Examiner |
|---|---|---|
| THE HAGUE | 10 FEBRUARY 1993 | LOISELET-TAISNE S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)